# EUROPEAN PATENT APPLICATION

(11) **EP 4 400 583 A1**
(43) Date of publication of application: **17.07.2024**
(21) Application number: 23151376.3
(22) Date of filing: 12.01.2023
(51) Int. Cl.: C12N 7/02, C12N 7/00

(54) **METHOD FOR GENERATING AND PURIFYING VIRAL VECTORS**

(71) Applicant: Sartorius Stedim Biotech GmbH, 37079 Göttingen (DE)
(72) Inventor: SABALLUS, Martin, 37249 Neu-Eichenberg (DE); KAMPMANN, Markus, 44149 Dortmund (DE)
(74) Representative: Gottschald Patentanwälte Partnerschaft mbB

(57) **Abstract**

The invention relates to a method for generating and purifying viral vectors (V), wherein the viral vectors (V) are generated by cells in a fluid (F) and wherein the fluid (F) comprising the viral vectors (V) is guided through a tangential flow filtration arrangement (2) for purification, whereby the viral vectors (V) are purified in the fluid (F). It is proposed that the cells and cell debris from the cells, which are contained in the fluid (F) comprising the viral vectors (V) after generating the viral vectors (V), are at least partially separated from the viral vectors (V) by a fluidized bed centrifuge (5) before purification by the tangential flow filtration arrangement (2).

## Description

The present invention relates to a method for generating and purifying viral vectors according to the general part of claim 1 and to a system for generating and purifying viral vectors according to the general part of claim 9.

The method in question may be applied in different kinds of bioprocesses. Exemplarily, the proposed method can be used in the area of cell and gene therapy or for vaccine development. Generally, the proposed method is not limited to a specific field but rather can be applied in various fields of biotechnology.

Generating viral vectors by cells is well known in the art. Depending on the nature of applied cells, different kinds of viral vectors can be generated, like retroviral vectors, including lentiviral vectors and gammaretroviral vectors, adenoviral vectors or adeno-associated viral vectors. Preferably, mammalian cell lines that are adapted for growth in suspension, such as HEK293 suspension cells or the like are used to produce the viral vectors. For this, cells are kept in a fluid, like a culture medium, under certain conditions, wherein the viral vectors are generated.

Thereafter, for example if a certain number of viral vectors is present, the fluid comprising the viral vectors and the cells as well as cell debris from the cells has to be purified to obtain the purified viral vectors that may then be used further. For purification, particularly for separating the cells and cell debris from the viral vectors, the fluid comprising the cells, cell debris and viral vectors might be guided through a filter arrangement for clarification, wherein the fluid passes through a filtering element, such as a membrane or the like. Here, larger impurities of a certain size, like cells and cell debris, are at least partly retained, wherein the fluid comprising the viral vectors as well as smaller impurities are passing through the filtering element. After filtering the fluid by such a "pass-through" filter arrangement, a tangential flow filtration arrangement is used to separate the remaining impurities from the viral vectors in the fluid and/or concentrate the viral vectors in the fluid. For this, the fluid comprising the viral vectors is guided for purification through a tangential flow filtration arrangement and the remained impurities are at least partly separated from the viral vectors accordingly. As a result, the viral vectors can be purified from larger and smaller impurities.

A known method for generating and purifying viral vectors in a fluid, that builds the basis of the invention, is disclosed in WO 2021/252782 A1. Here, a viral vector, like a lentiviral vector, is generated by cells in a fluid and the composition, which corresponds to the fluid comprising the viral vectors and cells as well as cell debris, is firstly fed through a filtration arrangement, in this case an alternating tangential flow filtration arrangement, wherein the fluid comprising the viral vectors passes through a filtering element of the filtration arrangement. Thereafter, the filtered fluid comprising the viral vectors is fed through a single-pass tangential flow filtration system (SPTFF system). As a result, larger impurities, such as cells and cell debris, are separated from the fluid within the filtration arrangement and smaller impurities are separated from the fluid thereafter. As a result, the viral vectors are purified in the fluid and thus represent a purified viral vectors composition.

Even though the known method allows already a good way for purification of generated viral vectors, the grade of purification is strongly impacted by the conditions, for example regarding the membrane, operating parameters, blocking and fouling of the membrane or the like, of the SPTFF system. Particularly over time, the separation performance of the SPTFF system might decrease due to fouling of the SPTFF system because of cells and cell debris blocking the membrane.

Beside this resulting decreasing separation performance of the SPTFF system, another problem of the known method is that by using a pass-through filter, the activity of the purified viral vectors in the fluid are decreasing rapidly, since a part of the viral vectors may be disrupted, when passing through the filter. Moreover, the viral vectors may be entrapped by the membrane and/or may adsorb to the membrane instead of passing through the membrane. Thus, the purified viral vectors composition, which is obtained, might only comprise a small amount of active viral vectors.

Therefore, there is still a need for further improvement of the known method.

The present invention is based on the problem of improving the known method such that the concentration and/or the activity of the purified viral vectors in the fluid can be increased.

The above-noted problem is solved by the features of the characterizing part of claim 1.

The main realization of the present invention is that at least parts of the cells and cell debris from the cells, which are inside the fluid after generating the viral vectors, are separated, particularly from the viral vectors, by a fluidized bed centrifuge before purification by the tangential flow filtration arrangement. The fluidized bed centrifuge is therefore arranged prior to the tangential flow filtration arrangement. The separation by the fluidized bed centrifuge may function as kind of a pretreatment step before the purification, namely separation and/or concentration, by the tangential flow filtration arrangement. As separation by the fluidized bed centrifuge can be adjusted extensively as demanded, particularly regarding rotational speed, flow velocity, duration of centrifugation and the like, a highly efficient as well as highly gentle separation is possible. In contrast to other centrifugation methods, separation, namely centrifugation, by the fluidized bed centrifuge thus leads to a higher activity of the purified viral vectors in the fluid. Furthermore, since the cells and the cell debris are separated from the fluid comprising the viral vectors by the fluidized bed centrifuge in a gentle way, further filtering, by using a pass-through filter, may be prevented, which leads to higher activities of the obtained viral vectors after the overall purification process. Also, it can be advantageously intended, to reuse at least a part of the cells, since these are intact, because of the gentle separation by the fluidized bed centrifuge. Overall, with the proposed method, the concentration as well as the activity of the viral vectors is increased.

In detail, it is proposed that the cells and cell debris, which are contained in the fluid comprising the viral vectors after generating the viral vectors, are at least partially separated, particularly from the viral vectors, by a fluidized bed centrifuge before purification by the tangential flow filtration arrangement. Thus, the concentration and activity of the purified viral vectors in the fluid can be increased.

According to claim 2, enzymes and, additionally or alternatively, charged beads may be added to the fluid prior to the fluidized bed centrifuge. Enzymes can degrade cells, particularly cell membranes, cell debris and/or DNA impurities such that for example the viscosity of the fluid is reduced and/or the molecular weight of proteins is reduced. Furthermore, if the viral vectors are not released by the cells into the fluid, degrading the cells, particularly the cells membrane or wall can lead to releasing the viral vectors into the fluid. Charged beads can also be used to improve the following separation process by the fluidized bed centrifuge, since at least a part of the cells, cell debris and/or DNA can bind on these. Since cells, cell debris and/or DNA impurities are bound to the charged beads and by separating the charged beads in the following separation process, the bound cells, cell debris and/or DNA are separated therewith.

Claim 3, advantageously, further specifies the separation by the fluidized bed centrifuge, wherein the separation comprises a loading phase, a washing phase and/or a discharging phase. During the loading phase, a chamber, particularly several chambers, of the fluidized bed centrifuge is loaded with the fluid. Preferably, loading is performed while the centrifugation already takes place. This way, the separation of the viral vectors from at least part of the cells, cell debris and/or DNA impurities, which are comprised in the fluid, advantageously already takes place. Within the washing phase, a wash fluid is passed through the chamber so that advantageously the cells, which are accumulated in the chamber can be washed. Hereby, remaining fluid might be washed out of the chamber by the wash fluid. It is furthermore possible that viral vectors, which remained in the chamber during the loading phase, are washed out with the fluid and/or the wash fluid during the washing phase. During the discharging phase, the cells, the cell debris and/or the DNA impurities that accumulated, in particular unilaterally, in the chamber of the fluidized bed centrifuge are discharged by a transport fluid such that these are washed out of the chamber particularly as waste or for reuse.

After separation, the fluid comprising the viral vectors may be guided from the fluidized bed centrifuge into a holding vessel according to claim 4. Particularly, if separation by the fluidized bed centrifuge is applied cyclically, the holding vessel provides an intermediate storage for the fluid. Further, for example, a constant outflow of fluid after the holding vessel can be provided or larger amounts of separated fluid can be stored.

According to claim 5, enzymes might be added to the fluid. Herewith, the cells, particularly the cells membrane and/or walls, the cell debris and/or the DNA impurities, which was or were not separated beforehand by the fluidized bed centrifuge during separation, can at least be partly degraded. Furthermore, the enzymes might degrade in a more efficient way since the cells, the cell debris and/or the DNA is or are already partly separated from the fluid such that the enzymes will only adsorb on cells, cell debris and/or DNA, which is or are not separated and is still comprised in the fluid. Furthermore, by degrading, the viscosity of the fluid may be reduced, which may simplify the following process steps. It is generally possible, to degrade at least part of the cells, the cell debris and/or the DNA impurities by adding enzymes before as well as additionally or alternatively after the fluidized bed centrifuge, which can improve the purification process in general. Degrading by enzymes might prevent the tangential flow filtration arrangement, particularly a single-pass tangential flow filter and/or a separating filter element of the tangential flow filtration arrangement, from blocking.

Claim 6 further specifies advantageously that the viral vectors are concentrated in the tangential flow filtration's retentate. Thus, impurities are separated and maintained in the fluid, which flows out as permeate. Herewith, an even more gentle way of purification is possible, since the viral vectors do not pass through a separating filter element, like a membrane, of the tangential flow filtration arrangement.

According to an embodiment of claim 7, an ion exchange arrangement is used for further purification of the viral vectors. The ion exchange arrangement enables to bind the viral vectors and thus obtain these from the fluid, wherein further impurities in the fluid are not bound and are separated from the viral vectors.

According to claim 8, the fluid may be cooled and/or heated within the different processing steps or rather components of the system. While regulating the temperature, generating and/or purification, particularly separation, of viral vectors can be optimized, particularly regarding the activity of the viral vectors.

A second teaching according to claim 9, which is of equal importance, relates to a system for generating and purifying viral vectors, in particular for performing the proposed method, wherein in particular the viral vectors are generated by cells in a fluid, comprising a tangential flow filtration arrangement for purification of the viral vectors generated by the cells in the fluid, wherein the system comprises a fluidized bed centrifuge for separating at least part of the cells and/or cell debris from the cells contained in the fluid after generating the viral vectors and prior to purification by the tangential flow filtration arrangement. The proposed system can be used for performing the proposed method. Therefore, all explanations given with regard to the proposed method are fully applicable. Furthermore, all following explanations with regard to the proposed system are fully applicable to the proposed method.

According to claim 10, the system may comprise an initial holding vessel. The initial holding vessel enables to receive and in particular temporarily hold a/the fluid. It may be intended that within the initial holding vessel the viral vectors are also generated by cells in the fluid. Particularly, if the initial holding vessel is arranged prior to the fluidized bed centrifuge. If the initial holding vessel is advantageously designed as a disposable initial holding vessel, contamination between different fluids with viral vectors can be avoided. Further, the sterility of the cell culture used to produce the viral vectors may be maintained. The initial holding vessel may comprise a mixing mean for mixing and thus homogenizing fluid received by the initial holding vessel.

Claim 11 and 12 further specify the fluidized bed centrifuge, particularly a chamber of the fluidized bed centrifuge. With such a chamber, the centrifugation may take place simultaneously to the fluid flowing through the chamber. A preferred embodiment according to claim 12 enables, particularly during the loading and/or washing phase, a higher inflow velocity at a first flow opening at the tip, where the centrifugal force acting on the fluid is greater, and enables a lower outflow velocity at a second flow opening at the base surface, where the centrifugal force acting on the fluid is lower, wherein as a result, the separation performance is improved.

The tangential flow filtration arrangement may comprise at least one single-pass tangential flow filter for the filtration of the fluid (claim 13). By passing the fluid through the filter arrangement in a single-pass, no recirculation of the fluid is required.

Furthermore, the system may comprise an ion exchange arrangement for purifying the viral vectors according to claim 14. As already described in context with the method, the ion exchange arrangement enables to bind the viral vectors and thus obtain these from the fluid, wherein further impurities in the fluid are not bound and are separated from the viral vectors.

In the following, embodiments of the invention are explained with respect to the drawing. The drawing shows in
- Fig. 1: a schematic representation of a proposed integrated system for purifying viral vectors produced by cells in a fluid,
- Fig. 2: a schematic representation of a further proposed integrated system, wherein the system comprises a holding vessel after the fluidized bed centrifuge
- Fig. 3: a schematic representation of different phases of the separation by the fluid-ized bed centrifuge, wherein a) shows a loading phase, b) shows a washing phase and c) shows a discharging phase,
- Fig. 4: a schematic representation of a further proposed integrated system, wherein the system comprises an advantageously embodiment regarding the tangential flow filtration arrangement,
- Fig. 5: a schematic representation of a further proposed integrated system, wherein the system comprises an advantageously embodiment regarding the tangential flow filtration arrangement, wherein the system comprising a holding vessel, and
- Fig. 6: a schematic representation of a further proposed integrated system, wherein during the process a diafiltration buffer is added to the fluid.

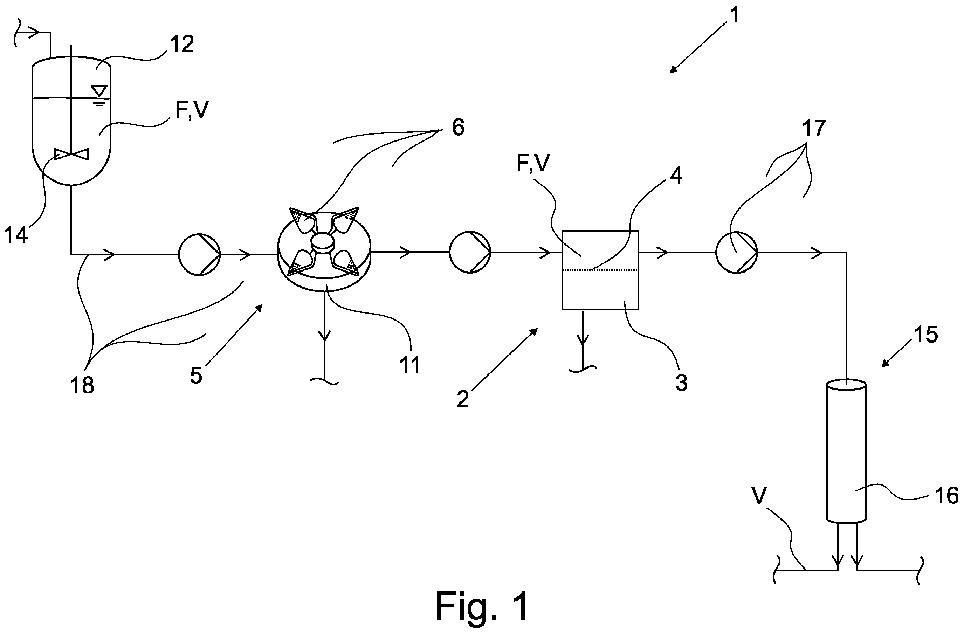

Proposed is a method for generating and purifying viral vectors V, in particular from and/or in a fluid F. The method can be performed with a system 1 as shown exemplarily in Fig. 1. The viral vectors V are generated by cells in the fluid F. For example, viral vectors V, like lentiviral vectors, adenoviral vectors, adeno-associated viral vectors, gammaretroviral vectors or the like may be generated by cells, like HEK-293 cells, in a fluid F, like a cell culture medium. Preferably, cells are growing in suspension in a serum-free culture medium and release the produced viral vectors into the culture medium. As shown exemplarily in Fig. 1, the viral vectors V may be generated within a holding vessel 12 of the system 1. The holding vessel 12 may be designed as a holding tank. Alternatively, the viral vectors V may be generated beforehand in the fluid F, for example in a batch process or the like, and may be guided to the system 1 thereafter.

The term "viral vectors" refers to all kinds of viruses. For example, viral vectors V may be virus-based tools that are used to transfer and integrate transgenes of interest into a target cell. Viral vectors may be produced by cells. Preferably, mammalian cells are used as cells to generate the viral vectors. It is further preferred that the cells are adapted to grow in suspension. In an especially preferred embodiment, suspension-adapted HEK293 cells are used to produce the viral vectors. For viral vector production, the cells are preferably maintained in a fluid F, such as a cultivation medium, and in a controlled culture environment. The term "cultivation medium" refers to specially designed growth media, which supply the nutrients required by the respective cells to produce the viral vectors. The term "culture environment" presently means the complex influence of the entirety of all physical, chemical and/or biological parameters, such as temperature, pH and nutrition concentrations, that act upon the cell or cells and determine its or their physiology and survival. The composition of the fluid F may be changing over time by the growth and the metabolism of the cells. Accordingly, the term "fluid" is to be understood in a broad sense and refers to all kinds of fluid, particularly liquids, such as suspensions, dispersions, etc. The composition of the fluid F may change during the process, for example because of the growth and metabolism of the cells and/or the ongoing separation and/or purification. The fluid F may comprise the cells, at least part of the ingredients of the cultivation medium (e.g., micro-nutrients, salts etc.), as well as components generated by the cells, before entering the fluidized bed centrifuge 5. The components generated by the cells may include the viral vectors, but may also include cell metabolites like lactate, cell debris and/or DNA.

"DNA" has to be understood broadly in the context of this application. DNA comprises particularly also chromatin and/or plasmid DNA from transfection and/or free nucleic acids.

"Cell debris" has to be understood broadly in the context of this application. Cell debris comprises particularly cells, which do not have an intact outer cell membrane or cell wall, particularly dead cells, cell fragments, such as parts of the cell membrane or cell wall and/or cell proteins, particularly cell proteins that are released from the cells when the cell membrane or cell wall is not intact.

The term "purifying" and "purification" in context with this application has to be understood in a broad sense. While purifying or during purification, impurities may be separated from the fluid F and/or the product and/or the product within the fluid F may be concentrated and/or the product may be separated from the fluid F. The product may be the viral vectors. Impurities may be cells and/or cell debris.

With the proposed method, the fluid F comprising the viral vectors V is for purification guided through a tangential flow filtration arrangement 2, whereby the viral vectors V are purified in the fluid F. Since, the viral vectors V are preferably the product, which shall be obtained, the tangential flow filtration arrangement 2 enables gently purification of the viral vectors V. By using the tangential flow filtration arrangement 2, impurities within the fluid F, like cells and/or cell debris, can be filtered out. As shown exemplarily in Fig. 1, the purified viral vectors V in the fluid F may stream out of the tangential flow filtration arrangement 2 as retentate R, thus the viral vectors V are partly, particularly mostly, hold back by the tangential flow filtration arrangement 2 and do not flow through any separating filter element 4 or the like of the tangential flow filtration arrangement 2. However, it is alternatively also possible that the fluid F comprising the viral vectors V may stream out of the tangential flow filtration arrangement 2 as permeate P, thus the viral vectors V may partly, particularly mostly, flow through the separating filter element 4 or the like of the tangential flow filtration arrangement 2. In context with the tangential flow filtration arrangement 2, the purification may comprise separating impurities from the viral vectors V and/or concentrating the viral vectors V in the fluid F. By using the tangential flow filtration arrangement 2, the purification thus may result in higher purities and/or higher concentrations of the viral vectors V.

The term "impurities" refers to any component present within the fluid that is not the product. Accordingly, components may be cells, cell debris, DNA, media ingredients and/or the like.

For the proposed method, it is essential that the cells and/or cell debris, which are contained in the fluid F comprising the viral vectors V after generating the viral vectors V, are at least partly separated, particularly from the viral vectors V, by a fluidized bed centrifuge 5 before purification by the tangential flow filtration arrangement 2. While generating the viral vectors V, the cells partly die, for example because of enzymes, which are added to the fluid F, or naturally. When dying, the cells may intracellular components, particularly proteins and/or DNA into the fluid. The cell debris comprise, inter alia, the dead cells. Due to separation by the fluidized bed centrifuge 5, a substantial part of the impurities within the fluid F, like cells and cell debris, can be separated before purification by the tangential flow filtration arrangement 2 and thus the overall purification can be improved.

In a preferred embodiment of the method and as shown in Fig. 1, for generating the viral vectors V, the fluid F is fed into a, particularly disposable, initial holding vessel 12. The fluid F may be the cultivation medium. Preferably, cells are added to the fluid F, preferably in the initial holding vessel 12 or beforehand, so that the viral vectors V are generated by the cells, in particular in the initial holding vessel 12. For homogenization reasons and as it is shown in Fig. 1, the fluid F in the initial holding vessel 12 is preferably mixed at least temporarily, for example by a mixing means 14 of the initial holding vessel 12. The mixing means 14 might be a mixer, like a dynamic mixer or the like. Further preferably, the initial holding vessel 12 comprises temperature means for controlling the temperature and/or aeration means for aeration of the fluid F in the initial holding vessel 12.

Preferably, prior to the fluidized bed centrifuge, preferably in the initial holding vessel 12, enzymes are added to the fluid F, so that the cells and/or the cell debris and/or the DNA impurities are degraded and/or reduced in molecular size. Degrading cells, cell debris and/or DNA by enzymes is common in the art, for example to reduce the viscosity of the fluid and/or to reduce the molecular weight of proteins. For degrading DNA, enzymes like nucleases, such as benzonase, may be used. Furthermore, particularly if the viral vectors V are not already transported to the fluid F by the cells, the viral vectors V preferably are released from the cells into the fluid F. Since, the viral vectors V preferably are the product, which shall be obtained by the overall purification process, the viral vectors V have to be released to the fluid F before separation of the cells and the cell debris by the fluidized bed centrifuge 5. Alternatively or additionally, charged beads CB are added to the fluid F, so that the cells, particularly the cell membranes or cell walls, and/or the cell debris and/or the DNA impurities are bound to the charged beads CB. The charged beads CB may improve the separation performance of the fluidized bed centrifuge 5, because the charged beads CB with the bound, namely adsorbed, cells and/or cell debris and/or DNA can be separated by the following separation, namely by the following centrifugation, more easily.

Within this application, the terms "prior to" or "after" a respective component of the system 1, like the fluidized bed centrifuge 5 or the like refer to the flow of fluid F and thus to the order, in which the fluid F flows through the respective components of the system 1.

Furthermore, it is possible that the fluid F comprising cells and/or cell debris is treated mechanically and/or via temperature shifting such that cells are lysed. Here, the cells may release the viral vectors V and/or the DNA inside the cells into the fluid F.

Alternatively or additionally, it is preferred that cell disrupting agents are added to the fluid F, particularly in the initial holding vessel 12 and/or an intermediate holding vessel 13. Here, the cells might be lysed such that particularly the viral vectors V and/or the DNA and/or other intracellular components like proteins and metabolites inside the cells are released into the fluid F. Cell disrupting agents might be detergents, like sodium dodecyl-sulfate, Triton X100, Tween, and/or enzymes, like lysozyme.

In a further preferred embodiment, the separation, particularly of the cells and/or cell debris and/or DNA impurities and/or cell debris from the viral vectors V, by the fluidized bed centrifuge 5 comprises a loading phase LP, in which the fluid F comprising the viral vectors V, cells and/or cell debris is passed through a chamber 6 of the fluidized bed centrifuge 5 during centrifugation, wherein at least a part of the cells and/or cell debris accumulates unilaterally in the chamber 6 due to the acting centrifugal force, particularly due to a balance between the fluid flow force and the centrifugal force. During the loading phase LP, the cells and/or cell debris may be separated at least partly from the fluid F comprising the viral vectors V.

Additionally, by using the fluidized bed centrifuge 5, there may be less shear stress exerted on the cells, which results in less cells losing their integrity and thus prevents the release of cell debris and/or DNA and/or other intracellular components into the fluid F.

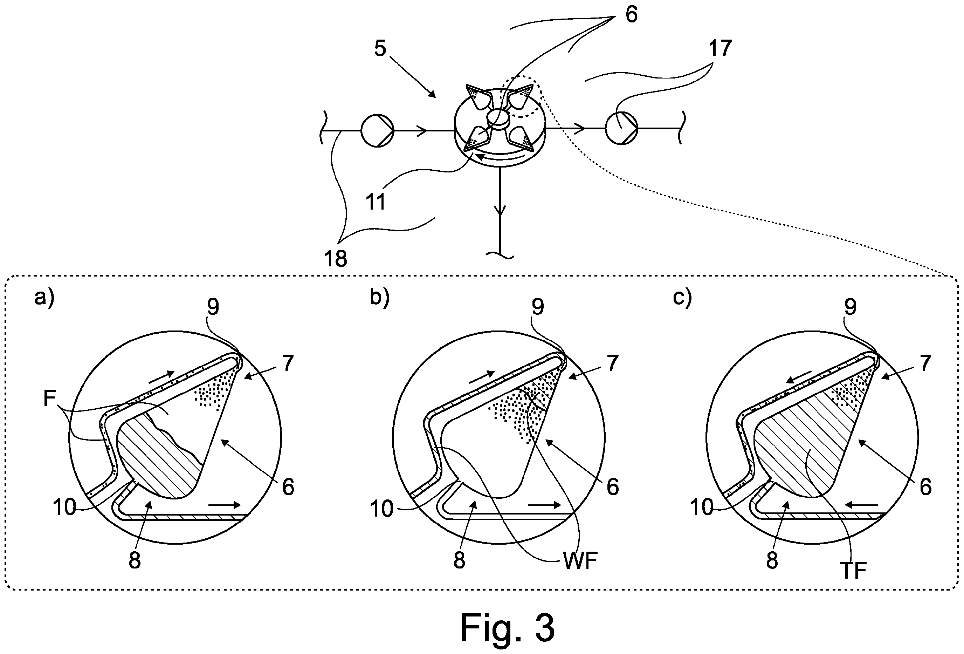

As exemplarily shown in Fig. 1 and 3, the fluidized bed centrifuge 5 comprises the chamber 6. The fluid F may be guided from the holding vessel 12 to the chamber 6 and passes through the chamber 6 during the loading phase LP, see Fig. 3a). The flow of the fluid F results in an acting flow force, which acts particularly on the viral vectors V and/or the cells and/or the cell debris, which is or are thus at least partly transported with the fluid F. Simultaneously, the chamber 6 rotates so that the centrifugal force is acting on the fluid F and particularly on the viral vectors V and/or the cells and/or the cell debris. Since, the acting flow force and the acting centrifugal force are acting in opposing directions, at least a part of the cells and/or cell debris accumulates unilaterally in the chamber 6 due to the acting centrifugal force, because the flow force, which acts on these, is respectively lower than the centrifugal force, which acts on these. Since, the flow force acting on the viral vectors V is larger than the centrifugal force acting on the viral vectors V, the viral vectors V are passing through the chamber 6 and being separated from at least part of the cells and/or the cell debris, which remain in the chamber 6. The usage of the charged beads CB, which are also separated by the fluidized bed centrifuge, can enhance this separation. The flow rate of the fluid F and/or the rotational speed of the fluidized bed centrifuge 5 may be adjusted. The flow rate of the fluid F and/or the rotational speed has preferably to be chosen such that the cells and/or cell debris at least partly, preferably mostly, remain in the chamber 6 during centrifugation, particularly during the loading phase and washing phase.

Preferably, the separation by the fluidized bed centrifuge 5 comprises a washing phase WP in which a wash fluid WF is passed through the chamber 6 during centrifugation, wherein the cells, which are accumulated in the chamber 6, are washed. By the washing phase WP, the separation can be optimized to that effect that any remaining fluid F, comprising the viral vectors V, as well as remaining further impurities, beside cells might be removed from the chamber 6. Beside that, it is possible that viral vectors V, which might be remained in the chamber 6, can be separated from at least a part of the cells and/or cell debris due to the acting flow force by the flow of the wash fluid WG through the chamber 6 and the wash fluid WF comprising the viral vectors V might flow out of the chamber 6. The viral vectors V, for example, may be hindered to pass through the chamber during the loading phase LP by the accumulating cells and/or cell debris and be washed out of the chamber 6 by the wash fluid WF during the washing phase WP. The wash fluid WF may be fluid F comprising viral vectors V or fluid F without viral vectors V. The wash fluid WF may subsequently to the fluidized bed centrifuge 5 be mixed with the fluid F, which passed through the chamber 6 during the loading phase, or may be purified further separately. The washing phase WP is exemplarily shown in Fig. 3b).

Alternatively or additionally, the separation by the fluidized bed centrifuge 5 comprises a discharging phase DP, in which a transport fluid TF is introduced into the chamber 6, wherein the cells and/or cell debris that are accumulated unilaterally in the chamber 6 flow out of the chamber with the transport fluid TF. During the discharging phase DP the cells and/or cell debris can be transported out of the chamber 6 with the transport fluid TF so that the chamber 6 is emptied. Since, the separation by using the fluidized bed centrifuge 5 is relatively gentle on the cells, the cells advantageously may be reused for further generating viral vectors V. The cells might be viable because of the gentle separation process by the fluidized bed centrifuge 5. Preferably, during the discharging phase DP, the transport fluid TF is introduced into the chamber 6 such that the direction of flow is opposite to the direction of flow of the fluid F during the loading phase LP and/or the wash fluid WF during the washing phase WP, like it is exemplarily shown in Fig. 3c). Further preferably, during the discharging phase DP, the chamber 6 rotates such that due to the acting centrifugal force, the emptying of the cells and/or cell debris is supported, like exemplarily shown in Fig. 3c).

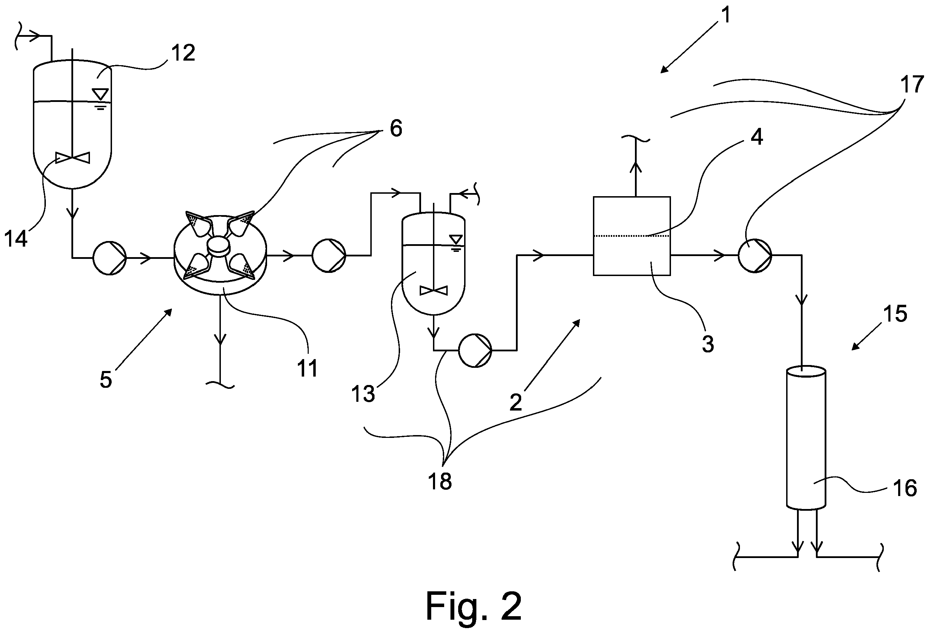

In a further preferred embodiment of the method, the fluid F comprising the viral vectors V is guided from the fluidized bed centrifuge 5 into an intermediate holding vessel 13, like it is exemplarily shown in Fig. 2. The intermediate holding vessel 13 enables to temporarily store the fluid F comprising the viral vectors V, which already passed the fluidized bed centrifuge 5. The intermediate holding vessel 13 may be designed as a holding tank. Preferably, the fluid F flows into the intermediate holding vessel 13 discontinuously and flows out of the intermediate holding vessel 13 continuously. Since, the separation by the fluidized bed centrifuge 5 may be discontinuous, the intermediate holding vessel 13 is preferably compensating the varying fluid flow from the fluidized bed centrifuge 5 such that the discontinuous outflowing fluid F, which flows out of the fluidized bed centrifuge 5, is temporarily stored and preferably a continuous flow of the fluid F out of the intermediate holding vessel 13 is enabled. Thus preferably, the fluid F comprising the viral vectors V flows discontinuously into the intermediate holding vessel 13 and flows continuously out of the intermediate holding vessel 13. Preferably, the intermediate holding vessel 13 is arranged after the fluidized bed centrifuge 5 and prior to the tangential flow filtration arrangement 2. It is possible to add components to the fluid F in the intermediate holding vessel 13, for example, like shown in Fig. 2, enzymes might be added.

It shall be noted that in context with this application the term "second" in combination with a respective component of the system 1 has not necessarily to be understood that the embodiment, particularly the system 1, does comprise at least two of the respective components. Moreover, the term "second" is chosen for distinction reason. For that reason, the system 1 may comprise a second component, but does not necessarily also comprise a respective first component.

According to a further preferred embodiment, enzymes are added to the fluid F, preferably after the fluidized bed centrifuge 5 and/or prior to the tangential flow filtration arrangement 2, so that the cells and/or the cell debris and/or the DNA impurities are degraded, in particular thereby releasing the viral vectors, and/or reduced in molecular size. As already described before, degrading cells, cell debris, particularly cell proteins, and/or DNA impurities by enzymes is common in the art. Adding enzymes to the fluid F after the fluidized bed centrifuge 5 and/or prior to the tangential flow filtration arrangement 2 can lead to a higher degradation performance, since the cells and/or cell debris are at least partly already separated from the fluid F. As a result, less enzymes may be needed to degrade the remaining cell debris and/or DNA impurities. Preferably, the enzymes might be added in an initial holding vessel 12 and/or an intermediate holding vessel 13.

It is preferred that the viral vectors V are concentrated in the fluid F, which flows out of the tangential flow filtration arrangement 2 as retentate R, and that impurities are present in the fluid, which flows out of the tangential flow filtration arrangement 2 as permeate P. Preferably, the viral vectors V are at least partly held back by the tangential flow filtration arrangement 2, in particular by the single-pass tangential flow filter 3 and/or the separating filter element 4 or the like. As a result, the viral vectors V, which are preferably the product of the overall purification process, do not pass through the separating filter element 4, for example a membrane, or the like and thus the activity of the viral vectors V leaving the tangential flow filtration arrangement 2 may be maintained. The viral vectors V are purified, since impurities are separated, namely filtered, from the viral vectors V and the concentration of viral vectors V in the fluid F, which flows out of the tangential flow filtration arrangement 2, raises. As it can be seen, for example, in Fig. 1, a stream of retentate R and a stream of permeate P leave the tangential flow filtration arrangement 2. The permeate P may comprise at least part of the impurities, particularly cells and/or cell debris and/or the DNA impurities. The retentate R may comprise the viral vectors V, such that the retentate R may further be purified after the tangential flow filtration arrangement 2.

For further purification, a preferred embodiment of the proposed method comprises that the fluid F comprising the viral vectors V is guided through an ion exchange arrangement 15, preferably after the tangential flow filtration arrangement 2, and the viral vectors V are purified. The ion exchange arrangement 15 preferably comprise an ion exchange material 16, preferably an anion exchange material, more preferably a weak anion exchange material. Alternatively, the ion exchange material 16 may be a weakly hydrophobic material. It is further preferred, that monoliths with a defined channel size distribution are used. In an especially preferred embodiment, the channel size is in the range of 6 µm. This allows larger particles, such as remaining cell debris, to pass through the ion exchange arrangement 15 instead of blocking the channels. In an exemplary embodiment, CIMmultus^{®} OH, CIMmultus^{®} SO3 or CIMmultus^{®} QA may be used. As, for example, Fig. 1 shows, the ion exchange arrangement 15 may be arranged after the tangential flow filtration arrangement 2. Here, the fluid F comprising the viral vectors V is guided from the tangential flow filtration arrangement 2 to the ion exchange arrangement 15. When the fluid F is flowing through the ion exchange arrangement 15, the viral vectors V are bound to the ion exchange material 16. Since, the impurities, which are still present in the fluid F, may have a different charge related to the viral vectors V, the impurities are, at least partly, not bound to the ion exchange material 16 and are thus separated from the viral vectors V. The impurities flow out of the ion exchange arrangement 15 with the fluid F, which flows through the ion exchange arrangement 15.

The viral vectors V, which are particularly the product of the overall process, can preferably be obtained as eluate, particularly by using a suitable elution reagent, wherein further preferably further fluid F, particularly without any impurities, with an adjusted pH and/or conductivity is guided through the ion exchange arrangement 15 and the viral vectors V are released from the ion exchange material 16. While in the embodiment of fig. 1, the viral vectors V are bound and the impurities are not bound to the ion exchange material 16 at first, it is also possible, that the impurities may be bound and the viral vectors V may not bound to the ion exchange material 16. This can be reached by adjustment of the pH, particularly a pH shift, of the fluid F before entering the ion exchange arrangement 15 and/or by selecting a different ion exchange material 16. In this case, the viral vectors V flow through the ion exchange arrangement 15 and the impurities can be eluted from the ion exchange arrangement 15.

It is possible that the pH and/or the conductivity of the fluid F is adjusted prior to the ion exchange arrangement 15. With the adjustment it may be regulated, if the viral vectors V bind to the ion exchange material 16 or if the impurities bind to the ion exchange material 16. It is preferred that the pH is adjusted such that the viral vectors V are bound to the ion exchange material 16 first and then are obtained in the fluid F as eluate. Preferably, the pH and/or conductivity adjustment may be performed by the tangential flow filtration arrangement 2, particularly in a single-pass tangential flow filter 3, so that the pH and/or the conductivity of the fluid F, which flows through the tangential flow filtration arrangement 2, is adjusted.

Preferably, the method comprises that the fluid F in the initial holding vessel 12 and/or in the fluidized bed centrifuge 5 and/or in an intermediate holding vessel 13 and/or in the tangential flow filtration arrangement 2 and/or in the ion exchange arrangement 15 and/or in fluid lines for guiding the fluid F is at least partially cooled and/or heated. Viral vectors V are often temperature sensitive. For that reason, at least partly regulating the temperature of the fluid F by heating or cooling within the process may lead to a higher activity of the purified viral vectors V and thus to a higher performance of the process. Preferably, the temperature of the fluid F is set between 4°C and 37 °C, more preferably, the temperature of the fluid F is set to 4°C.

Beside the above-noted method, as a second teaching, the system 1 is described in context with the drawing. As the above-noted method can be performed with the proposed system 1, all explanations made and features of the method are adaptable to the system 1 and vice versa.

The system 1 for purifying viral vectors V generated by cells in a fluid F comprises a tangential flow filtration arrangement 2 for purification of the viral vectors V generated by the cells in the fluid F. As it can be seen in Fig. 1, 2, 4, 5 and 6 and preferably, the system 1 comprises, which is essential, a fluidized bed centrifuge 5 for separating the cells and/or cell debris contained in the fluid F after generating the viral vectors V and prior to purification by the tangential flow filtration arrangement 2. The fluidized bed centrifuge 5 is located prior to the tangential flow filtration arrangement 2 such that the fluid F comprising the viral vectors V can flow through the fluidized bed centrifuge 5 first and through the tangential flow filtration arrangement 2 thereafter. Since, impurities, like cells and cell debris, are already partly separated by the fluidized bed centrifuge 5, the overall performance of the purification process can be improved.

Generally, the performance of the separation process by the fluidized bed centrifuge 5 may be increased by adjustment of the flow rate through the fluidized bed centrifuge 5, wherein low flow rates may lead to higher performances, particularly as smaller particles are retained within the chamber 6 compared to when higher flow rates are utilized. Furthermore, the performance of the purification process, namely separation process, by the tangential flow filtration arrangement 2 may be increased by adjustment of the flow rate through the tangential flow filtration arrangement 2, wherein low flow rates may lead to higher performances. The increase of separation performance may lead to an increase of the overall purification performance.

According to a preferred embodiment, the system 1 comprises an initial holding vessel 12. The initial holding vessel 12 may enable to generate viral vectors V in the fluid F. The fluid F may be the cultivation medium. Preferably, cells are added to the fluid F within the initial holding vessel 12 or before guiding the fluid F into the initial holding vessel 12. Alternatively, fluid F, which already comprises viral vectors V, might be filled into the initial holding vessel and is temporarily stored in the initial holding vessel 12. Preferably, the initial holding vessel 12 is designed as a disposable initial holding vessel 12. Thus, the risk of contamination can be reduced, because for every run of viral vectors V, which are purified, the initial holding vessel 12 might be changed within the system 1. Further preferably, the initial holding vessel 12 comprises at least one mixing means 14 for mixing fluid F received by the initial holding vessel 12. Here, the fluid F is homogenized, what may result in higher generation rates of viral vectors V and/or in a higher activity of the purified viral vectors V and/or in a higher overall performance of the purification process. It is possible to add enzymes and/or charged beads CB to the fluid F in the initial holding vessel 12.

In a further preferred embodiment, the system 1 comprises an intermediate holding vessel 13. The intermediate holding vessel 13 may buffer discontinuous flow of fluid F from the fluidized bed centrifuge 5. Furthermore, it is possible to add enzymes and/or charged beads CB to the fluid F in the intermediate holding vessel 13. The intermediate holding vessel 13 might comprise mixing means 14 for homogenization. Fig. 2 shows exemplarily an embodiment of system 1 comprising the intermediate holding vessel 13.

Advantageously, the fluidized bed centrifuge 5 comprise at least one chamber 6 through which the fluid F to be centrifuged can flow, as particularly shown in Fig. 3a, 3b and 3c. As described before in context with the method, the chamber 6 of the fluidized bed centrifuge 5 is flowed through by fluid F comprising the viral vectors V and impurities, wherein simultaneously the fluid F is centrifuged, particularly such that at least part of the impurities are separated from the fluid F.

With regard to the design of the chamber 6, it is preferred if the chamber 6 is conical with a tip 7 and a base surface 8 facing the tip 7, as shown particularly in Fig. 3a, 3b and 3c. The conical design of the chamber 6 enables to simply regulate the flow of the fluid F through the chamber 6 during centrifugation, because of a changing cross section, particularly such that the fluid flow velocity is faster close to the tip 7 compared to the fluid flow velocity close to the base surface 8. Preferably, a first flow opening 9 for the inflow or outflow of fluid F into or out of the chamber 6 is arranged at the tip 7 of the chamber 6. Additionally or alternatively, a second flow opening 10 for the inflow or outflow of fluid F into or out of the chamber 6 is arranged at the base surface 8 of the chamber 6. The first flow opening 9 and/or the second flow opening 10 may have a double function as inflow and outflow opening. For example, fluid F may enter the chamber 6 during the loading phase LP, as shown in Fig. 3a. Here, the fluid F comprising the viral vectors V is flowing into the chamber 6 via the first flow opening 9. Furthermore, the fluid F is flowing out of the chamber 6 via the second flow opening 10. Thus, the first flow opening 9 enables inflow into the chamber 6 and the second flow opening 10 enables outflow out of the chamber 6. During the washing phase WP, wash fluid WF may flow into the chamber 6 via the first flow opening 9 and out of the chamber 6 via the second flow opening 10. During the discharging phase DP, the transport fluid TF may flow out of the chamber 6 via the first flow opening 9 and into the chamber 6 via the second flow opening 10, since the flow direction of the transport fluid TF may be opposed to the flow direction of the fluid F and/or the wash fluid WF.

According to a further preferred embodiment, the chamber 6 is arranged on a rotating means 11, which rotates during centrifugation, such that during centrifugation of fluid, particularly fluid F, wash fluid WF and/or transport fluid TF, by the fluidized bed centrifuge 5, the centrifugal force acting on the fluid, and particularly on the impurities, is greater at the tip 7 of the chamber 6 than at the base surface 8. Hereby, the performance of the centrifugation can be improved, particularly because of a higher acting centrifugal force at the tip 7, where the fluid flow may be higher, than at the base surface 8, where the fluid flow may be lower. As a result of the fluid flow velocity gradient and the centrifugal force gradient formed within the chamber 6, particles with a higher density and/or a lager diameter accumulate near the tip 7 while particles with a lower density and/or a smaller diameter may accumulate towards the base surface 8. It is thus possible that the retention of particles with a certain size and/or density within the chamber 6 can be controlled by the rotation speed and/or the flow rate of the fluid F.

Advantageously, the tangential flow filtration arrangement 2 comprises at least one single-pass tangential flow filter 3 for the filtration of the fluid F, as it can exemplarily be seen in Fig. 1. The single-pass tangential flow filter 3 may act as a pretreatment before the fluid F enters an ion exchange arrangement 15. The single-pass tangential flow filter 3 may comprise a separating filter element 4, like a membrane or the like, which acts as a filtering element of the single-pass tangential flow filter 3. Preferably, the separating filter element 4 is designed such that the molecular weight cut-off is at least 500 kDa, preferably at least 400 kDa, further preferably at least 300 kDa ("kDa", kilodalton, 1 Da = 1 g/mol).

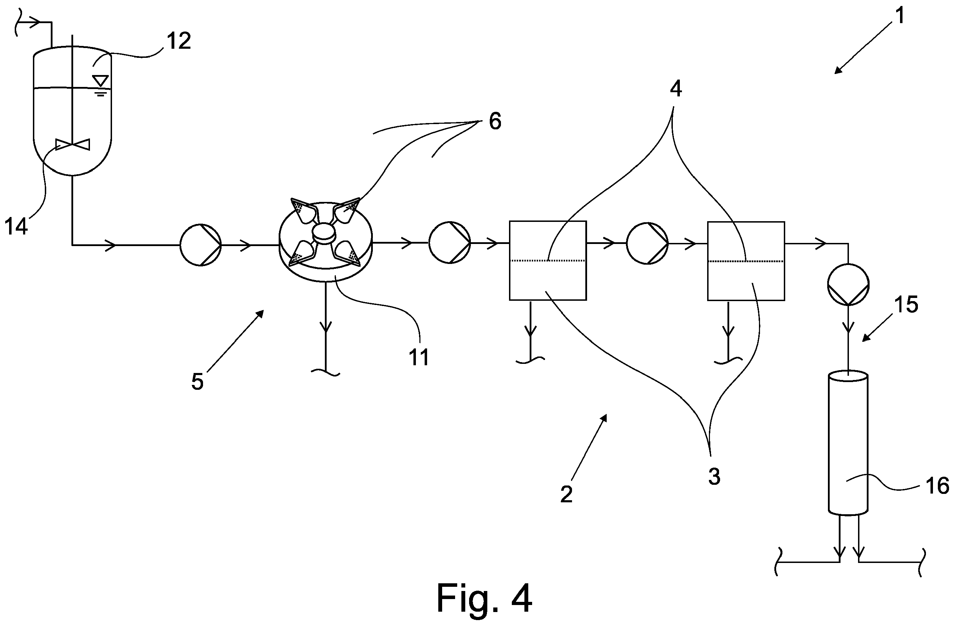

It is also possible and preferred that the tangential flow filtration arrangement 2 comprises two single-pass tangential flow filters 3, as it can, for example, be seen in Fig. 4. Here and preferred, the single-pass tangential flow filters 3 are arranged in series. The separation, namely the filtration, by each single-pass tangential flow filter 3 might be under different conditions such that the filtration process might be regulated. Furthermore, using more than one single-pass tangential flow filter 3 might lead to higher purification performance, like higher separation performance and/or higher concentration of viral vectors V after the tangential flow filtration arrangement 2.

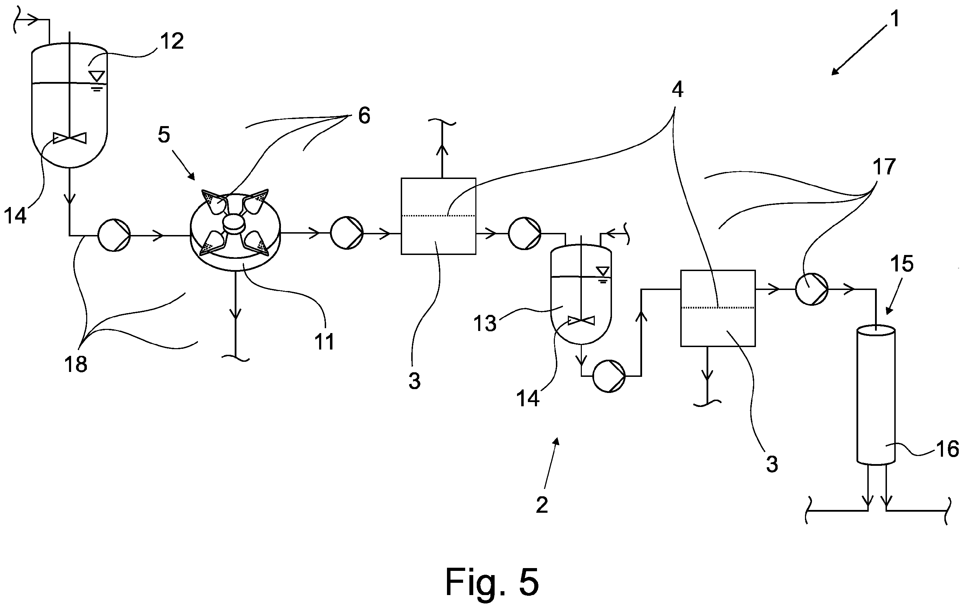

As shown in the embodiment of Fig. 5 and preferred, the pH and/or the conductivity of the fluid F may be adjusted, particularly shifted, after separation by the single-pass tangential flow filter 3 before separation by the following single-pass tangential flow filter 3. Here and preferably, the pH and/or the conductivity may be adjusted, particularly by adding a buffer solution, inside the intermediate holding vessel 13, which is arranged between the single-pass tangential flow filters 3. Adjustment of the pH and/or the conductivity might lead to improved conditions in the subsequent ion exchange arrangement 15.

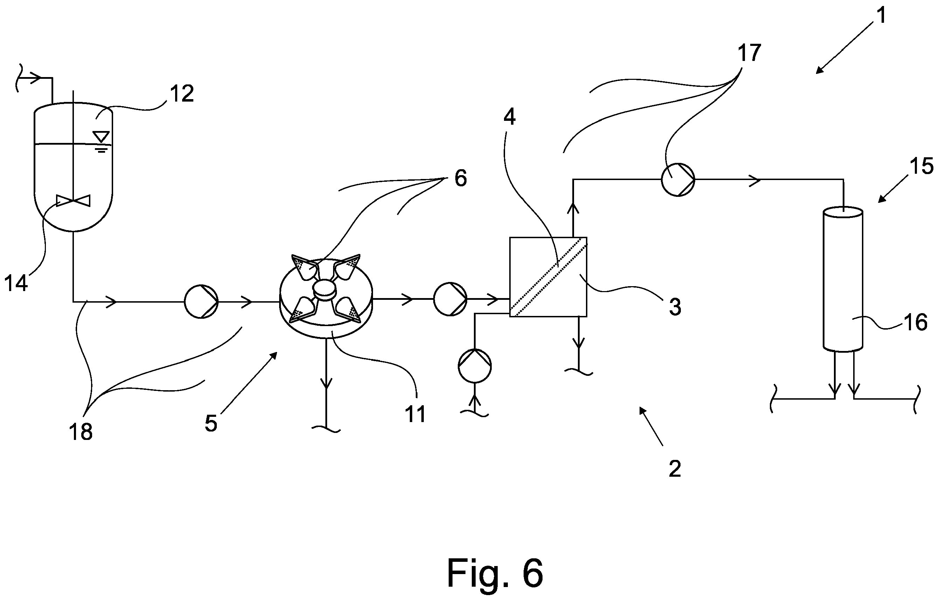

According to another preferred embodiment, exemplarily shown in Fig. 6, the pH and/or conductivity of the fluid F may be adjusted, particularly shifted, during separation by the single-pass tangential flow filter 3. Here, a buffer solution is directly added to the fluid F, when flowing through the single-pass tangential flow filter 3. The single-pass tangential flow filter 3 is preferably designed such that a buffer solution can be added to the fluid F during the purification, particularly the separation, by the single-pass tangential flow filter 3.

In a further advantageous embodiment, the system 1, particularly the tangential flow filtration arrangement 2, comprises a first pre-filter, which is arranged prior to the single-pass tangential flow filter 3 and/or prior to the separating filter element 4. Because of the first pre-filter, blocking of the single-pass tangential flow filter 3 and/or of the separating filter element 4 can be avoided, since larger impurities may be filtered out by the first pre-filter. The first pre-filter might be designed as a safety pre-filter.

Furthermore, it may possible that the system 1, particularly the ion exchange arrangement 15, comprises a second pre-filter, which is arranged prior to the ion exchange material 16. Because of the second pre-filter, negative effects, like blocking, of the ion exchange material 16 can be avoided, since larger impurities may be filtered out by the second pre-filter. The second pre-filter might be designed as a safety pre-filter.

Furthermore, it is preferred, that the system 1 comprises an ion exchange arrangement 15 for purifying the viral vectors V. As described in context with the method, the ion exchange arrangement 15 enables further purification of the viral vectors V, wherein the viral vectors V may be obtained directly with the flow-through fluid F or as eluate. Preferably, the ion exchange arrangement 15 comprises at least one ion exchange material 16. Here, anion exchange material as well as cation exchange material are generally possible. It is further preferred that the ion exchange material 16 is an ion, particularly an anion or cation, exchange resin, which is particularly arranged in a column or formed as a membrane. The ion exchange arrangement 15 preferably comprise an ion exchange material 16, preferably an anion exchange material, more preferably a weak anion exchange material. Alternatively, the ion exchange material 16 may be a weakly hydrophobic material. It is further preferred, that monoliths with a defined channel size distribution are used. In an especially preferred embodiment, the channel size is in the range of 6 µm. This allows larger particles, such as remaining cell debris, to pass through the ion exchange arrangement 15 instead of blocking the channels. In an exemplary embodiment, CIMmultus^{®} OH, CIMmultus^{®} SO3 or CIMmultus^{®} QA may be used.

It is further preferred that the system 1 comprises at least one pumping device 17. The pumping device 17 or the pumping devices 17 may be designed respectively as a peristaltic pump. Preferably, the system 1 comprises several pumping devices 17, which are arranged respectively prior to the fluidized bed centrifuge 5 and/or after the fluidized bed centrifuge 5 and prior to the tangential flow filtration arrangement 2 and/or after the tangential flow filtration arrangement 2 and prior to the ion exchange arrangement 15, as is exemplarily shown in Fig. 1.

Preferably, the system 1 comprises fluid lines 18 for guiding the fluid F within the system 1. Preferably, the fluid lines 18 are arranged such that fluid F can be guided from and to the components of the system 1, like the fluidized bed centrifuge 5 and/or the tangential flow filtration arrangement 2 and/or the ion exchange arrangement 15 and/or the initial holding vessel 12 and/or the intermediate holding vessel 13. Further preferably, the fluid lines 18 are respectively designed as tubes and/or pipes. Further preferably, if the pumping device 17 or the pumping devices 17 are designed as peristaltic pump or peristaltic pumps, the fluid lines 18 may be designed as tubes and/or pipes.

Within the figures of the drawing, the direction of flow of fluid F is generally from left to right. As it can be seen exemplarily in Fig. 1, within the system 1, the fluidized bed centrifuge 5 is arranged, regarding the flow of the fluid F, prior to the tangential flow filtration arrangement 2. Thus, the fluid F is entering the fluidized bed centrifuge 5 before the tangential flow filtration arrangement 2. In other words, separation by the fluidized bed centrifuge 5 happens prior to purification, namely separation, like filtration, and/or concentrating, by the tangential flow filtration arrangement 2.

Furthermore, as it can be seen in Fig. 2, further components, like an intermediate holding vessel 13, of the system 1 might be arranged between the fluidized bed centrifuge 5 and the tangential flow filtration arrangement 2. As it further can be seen exemplarily in Fig. 1, within the system 1, the tangential flow filtration arrangement 2 is preferably arranged, regarding the flow of the fluid F, prior to the ion exchange arrangement 15. Thus, the fluid F is entering the tangential flow filtration arrangement 2 before the ion exchange arrangement 15. It is possible that further components, like a further holding vessel or the like, of the system 1 might be arranged between the tangential flow filtration arrangement 2 and the ion exchange arrangement 15. Prior to the fluidized bed centrifuge 5, the initial holding vessel 12 might be preferably arranged, like it is shown, for example, in Fig. 1. The fluid F might enter the system 1 in the initial holding vessel 12.

## Claims

1. Method for generating and purifying viral vectors (V), wherein the viral vectors (V) are generated by cells in a fluid (F) and wherein the fluid (F) comprising the viral vectors (V) is guided through a tangential flow filtration arrangement (2) for purification, whereby the viral vectors (V) are purified in the fluid (F),
**characterized in**
**that** the cells and cell debris from the cells, which are contained in the fluid (F) comprising the viral vectors (V) after generating the viral vectors (V), are at least partially separated from the viral vectors (V) by a fluidized bed centrifuge (5) before purification by the tangential flow filtration arrangement (2).

2. Method according to claim 1, **characterized in that** prior to the fluidized bed centrifuge (5), wherein enzymes are added to the fluid (F), so that the cells and/or the cell debris and/or the DNA impurities are degraded, wherein in particular the viral vectors (V) are released from the cells into the fluid (F), and/or reduced in molecular size, and/or that charged beads (CB) are added to the fluid (F), so that the cells and/or cell debris and/or the DNA impurities are bound to the charged beads (CB).

3. Method according to claim 1 or 2, **characterized in that** the separation by the fluidized bed centrifuge (5) comprises a loading phase (LP), in which the fluid (F) comprising the viral vectors (V), cells and/or cell debris is passed through a chamber (6) of the fluidized bed centrifuge (5) during centrifugation, wherein at least a part of the cells and/or cell debris accumulate unilaterally in the chamber (6) due to the acting centrifugal force, and/or,
that the separation by the fluidized bed centrifuge (5) comprises a washing phase (WP) in which a wash fluid (WF) is passed through the chamber (6) during centrifugation, wherein the cells, which are accumulated in the chamber (6), are washed, and/or,
that the separation by the fluidized bed centrifuge (6) comprises a discharging phase (DP), in which a transport fluid (TF) is introduced into the chamber (6), wherein the cells and/or the cell debris that are accumulated unilaterally in the chamber (6) flow out of the chamber (6) with the transport fluid (TF).

4. Method according to one of the previous claims, **characterized in that** the fluid (F) comprising the viral vectors (V) is guided from the fluidized bed centrifuge (5) into an intermediate holding vessel (13), preferably **in that** the fluid (F) flows into the intermediate holding vessel (13) discontinuously and flows out of the intermediate holding vessel (13) continuously.

5. Method according to one of the previous claims, **characterized in that** enzymes are added to the fluid (F) so that the cells and/or the cell debris and/or the DNA impurities are degraded, in particular thereby releasing the viral vectors (V), and/or reduced in molecular size, preferably, that the enzymes are added to the fluid after the fluidized bed centrifuge (5) and/or prior to the tangential flow filtration arrangement (2).

6. Method according to one of the previous claims, **characterized in that** the viral vectors (V) are concentrated in the fluid (F), which flows out of the tangential flow filtration arrangement (2) as retentate (R) and impurities are separated into the fluid, which flows out of the tangential flow filtration arrangement (2) as permeate (P).

7. Method according to one of the previous claims, **characterized in that** the fluid (F) comprising the viral vectors (V) is guided through an ion exchange arrangement (15), preferably after the tangential flow filtration arrangement (2), and the viral vectors (V) are purified.

8. Method according to one of the previous claims, **characterized in that** the fluid (F) in a initial holding vessel (12) and/or in the fluidized bed centrifuge (5) and/or in the intermediate holding vessel (13) and/or in the tangential flow filtration arrangement (2) and/or in the ion exchange arrangement (15) and/or in fluid lines (18) for guiding the fluid (F) is at least partially cooled and/or heated.

9. System for generating and purifying viral vectors (V), in particular for performing the method according to one of the previous claims, comprising a tangential flow filtration arrangement (2) for purification of the viral vectors (V) generated by cells in a fluid (F),
**characterized in**
**that** the system (1) comprises a fluidized bed centrifuge (2) for separating the cells and/or cell debris from the cells contained in the fluid (F) after generating the viral vectors (V) and prior to purification by the tangential flow filtration arrangement (2).

10. System according to claim 9, **characterized in that** the system (1) comprises an initial holding vessel (12), which is preferably arranged prior to the fluidized bed centrifuge (5), preferably, that the initial holding vessel (12) is designed as a disposable initial holding vessel, further preferably, that the initial holding vessel (12) has at least one mixing means (14) for mixing fluid received by the initial holding vessel (12), and/or,
that the system (1) comprises an intermediate holding vessel (13), which is preferably arranged after the fluidized bed centrifuge (5), preferably, that the intermediate holding vessel (13) is designed as a disposable intermediate holding vessel, further preferably, that the intermediate holding vessel (13) has at least one mixing means (14) for mixing fluid received by the intermediate holding vessel (13).

11. System according to claim 9 or 10, **characterized in that** the fluidized bed centrifuge (5) has at least one chamber (6), preferably four chambers (6), through which fluid (F) to be centrifuged can flow.

12. System according to claim 11, **characterized in that** the chamber (6) is conical with a tip (7) and a base surface (8) facing the tip (7), preferably, that a first flow opening (9) for the inflow or outflow of fluid (F) into or out of the chamber (6) is arranged at the tip (7) of the chamber (6) and/or a second flow opening (10) for the inflow or outflow of fluid (F) into or out of the chamber (6) is arranged at the base surface (8) of the chamber (6), preferably, that the chamber (6) is arranged on a rotating means (11), which rotates during centrifugation, such that during centrifugation of fluid (F) by the fluidized bed centrifuge (5) the centrifugal force acting on the fluid is greater at the tip (7) of the chamber (8) than at the base surface (8).

13. System according to one of the claims 9 to 12, **characterized in that** the tangential flow filtration arrangement (2) comprises at least one single-pass tangential flow filter (3) for the filtration of the fluid (F).

14. System according to one of the claims 9 to 13, **characterized in that** the system (1) comprises an ion exchange arrangement (15) for purifying the viral vectors (V), preferably, that the ion exchange arrangement (15) comprises at least one ion exchange material (16), in particular an ion exchange resin or an ion exchange monolith, wherein the ion exchange resin is composed of a porous matrix having the form of spherical particles or a polymer membrane, preferably, that the ion exchange material (16) is formed as an anion exchange material, in particular as an anion exchange resin or an anion exchange monolith, wherein the anion exchange resin is composed of a porous matrix having the form of spherical particles or polymer membrane.
